# EUROPEAN PATENT SPECIFICATION

(11) **EP 1 630 553 B1**
(45) Date of publication and mention of the grant of the patent: **11.12.2013**
(21) Application number: 05017793.0
(22) Date of filing: 16.08.2005
(51) Int. Cl.: G01N 1/22, G01N 33/00

(54) **Gas sensor unit**
Gassensoreinheit
Unité de capteur de gaz

(30) Priority: 19.08.2004 JP 2004239055
(43) Date of publication of application: 01.03.2006
(73) Proprietor: NGK SPARK PLUG CO., LTD., Nagoya-shi, Aichi 467-8525 (JP)
(72) Inventor: Osawa, Norimasa c/o NGK Spark Plug Co.,Ltd., Nagoya, Aichi (JP); Hirai, Kazuto c/o NGK Spark Plug Co.,Ltd., Nagoya, Aichi (JP); Matsuoka, Toshiya c/o NGK Spark Plug Co.,Ltd., Nagoya, Aichi (JP)
(74) Representative: Zimmermann, Gerd Heinrich

(56) References cited:
- EP-A- 0 712 745
- DE-U1-202004 004 808
- US-A1- 2003 235 516
- PATENT ABSTRACTS OF JAPAN vol. 2000, no. 20, 10 July 2001 (2001-07-10) & JP 2001 066282 A (NGK SPARK PLUG CO LTD), 16 March 2001 (2001-03-16)
- PATENT ABSTRACTS OF JAPAN vol. 2003, no. 02, 5 February 2003 (2003-02-05) & JP 2002 318216 A (NGK SPARK PLUG CO LTD), 31 October 2002 (2002-10-31)
- PATENT ABSTRACTS OF JAPAN vol. 2000, no. 05, 14 September 2000 (2000-09-14) & JP 2000 043547 A (CALSONIC CORP), 15 February 2000 (2000-02-15)
- PATENT ABSTRACTS OF JAPAN vol. 2003, no. 10, 8 October 2003 (2003-10-08) & JP 2003 161712 A (RIKEN KEIKI CO LTD), 6 June 2003 (2003-06-06)

## Description

The present invention relates to a gas sensor unit. The present invention relates particularly to a gas sensor unit having a casing in which a gas sensor element is accommodated.

Conventionally, there have been proposed various gas sensor units each having a casing in which a gas sensor element is accommodated. An example of such a gas sensor unit is a gas sensor unit attached to the body of an automobile or the like or to a duct of an air conditioner for a vehicle and adapted to detect a change in concentration of a specific gas (NOₓ, HC, CO, etc.) contained in exhaust gas in external air (for example, see Japanese Patent Application Laid-Open (*kokai*) No. H9-184817, henceforth referred to as D1 and Japanese Patent Application Laid-Open (*kokai*) No. 2002-318217, henceforth referred to as D2). Specifically, such a gas sensor unit is used in a mechanism which automatically switches a flap between open and close positions when the concentration change of the specific gas in the external air becomes equal to or higher than a predetermined level, to thereby control the operation mode between the internal air circulation mode and the external air introduction mode.

In the gas sensor units disclosed in D1 and D2, an air communication hole (introduction passage) for introducing external air into the interior of the casing is formed in the casing (in D1, in a case cap fixed to the casing). The external air introduced via the air communication hole (introduction passage) is led to a gas sensor element so as to detect a change in concentration of a specific gas contained in the external air. Further, in the gas sensor units disclosed in D1 and D2, a filter having gas permeability and water repellency is provided in a gas flow passage within the casing to be located between the introduction passage and the gas sensor element so as to prevent water having entered the casing from the introduction passage from reaching the gas sensor element.

In recent years, a high-pressure washing machine which injects pressurized water from a nozzle at high speed has been widely used so as to wash automobiles or the like. When an automobile is washed by use of such a high-pressure washing machine, in some cases, water enters into the interior of the casing (into the interior of the case cap in D1) of a gas sensor unit from the introduction passage thereof, and then passes through the filter having water repellency and reaches the gas sensor element, whereby an anomaly arises in the gas sensor unit. As described above, in the case of the gas sensor units disclosed in Patent D1 and D2, there is a possibility that when water enters at high speed via the introduction passage, such water passes through the filter having water repellency and reaches the gas sensor element.

In order to suppress permeation of water entering at high speed, the withstand water pressure of the filter may be increased. However, when the withstand water pressure of the filter is increased, the gas permeability of the filter is lowered, thereby lowering the responsiveness of the gas sensor. Accordingly, the method of increasing the withstand water pressure of the filter has an inherent limit.

Further, when a foreign object (e.g., sand particles) flies from the outside, the foreign object may enter the interior of the casing via the introduction passage, and hit the water repellant filter, possibly resulting in breakage of the water repellant filter. Moreover, when an operator strongly inserts an attachment tool or the like into the interior of the casing via the introduction passage, the filter may be damaged by the attachment tool or the like.

A gas sensor which can maintain a high waterproofing property and high air permeability is disclosed in JP 2001 066282A. The gas sensor includes a lower-stage shielding plate and an upper-stage shielding plate, which are arranged and installed in such that a bent air flow path is defined.

US 2003-235516 A1 discloses a gas detector which includes a first shielding plate to prevent water entry.

A further gas sensor is described in JP 2002 318216A. A sensor element is provided within a housing which includes introduction passages at an upper part of the housing. Claim 1 is delimited with respect to this document.

EP 0 712 745 A1 describes another gas sensor with an air passage having an air inlet and an air outlet located above the air inlet. A filter is provided in the air passage below and downstream of a gas sensor. A labyrinth structure may be provided in the air passage between the air inlet and the filter for causing the air to meander at the labyrinth structure.

The present invention has been accomplished in view of the above problems. It is the object of the present invention to provide a gas sensor unit which overcomes at least some of the problems in the art. It is particularly an object of the present invention to provide a gas sensor unit which secures high responsiveness of a gas sensor, reduces the possibility that water having entered from the outside via an introduction passage passes through a filter, and/or reduces the possibility that the filter is damaged by a foreign object or the like having entered from the outside via the introduction passage.

The object is solved by a gas sensor unit according to claim 1. Further advantages, features, aspects and details of the invention are evident from the dependent claims, the description and the accompanying drawings.

According to one aspect of the present invention, the means for solution is a gas sensor unit comprising a gas sensor element for detecting changes in concentration of a specific gas contained in ambient gas; a casing for accommodating the gas sensor element, and forming a gas flow passage for leading to the gas sensor element the ambient gas introduced from the outside of the casing; and a filter having gas permeability and water repellency and water-tightly closing the gas flow passage at the middle thereof while allowing passage of gas. The gas flow passage includes a plurality of introduction passages for introducing the ambient gas from the outside of the casing, and a pre-filter passage which is located between the introduction passages and the filter and which the filter faces. The plurality of introduction passages are disposed at respective positions such that each introduction passage enables viewing of at least a portion of another introduction passage from the outside of the casing via the former introduction passage and the pre-filter passage. The filter is disposed at a position which cannot be viewed from the outside of the casing via any of the introduction passages.

In the following, several preferred aspects and embodiments of the invention are presented.

In a preferred embodiment of the gas sensor unit of the present invention, the plurality of introduction passages are disposed at respective positions such that each introduction passage enables viewing of at least a portion of another introduction passage from the outside of the casing via the former introduction passage and the pre-filter passage. By virtue of this structure, even when water enters from the outside at high speed via a certain introduction passage, the water having entered can be discharged to the outside via any one of the remaining introduction passages. Specifically, when a direction in which one can view, from the outside of the gas sensor unit, a certain introduction passage (first introduction passage) and can view another introduction passage (second introduction passage) via the pre-filter passage is considered to be a 1-2 view direction, water having entered at high speed from the outside via the first introduction passage in the 1-2 view direction can be discharged to the outside via the second introduction passage. Accordingly, water having entered the gas flow passage via the introduction passages can be prevented from remaining within the pre-filter passage, and it becomes possible to reduce the possibility that the pressure of water exceeds the withstand water pressure of the filter and the water passes through the filter.

In addition, since the introduction passages are disposed at respective positions as described above, the ambient gas entering the gas flow passage via the introduction passages is quickly led to the pre-filter passage regardless of the position of the introduction passage through which the ambient gas enters. Moreover, the pre-filter passage is disposed such that the filter faces the pre-filter passage. In other words, the filter is provided at a location where the filter faces the pre-filter passage, and nothing is disposed between the filter and the pre-filter passage. Therefore, as compared with the case where, in order to prevent entry of water, the pre-filter passage is bent to form a maze-like flow path, the atmospheric gas having entered from the outside can be quickly led from the pre-filter passage to the gas sensor element via the filter, so that the gas sensor can have excellent responsiveness.

Moreover, in the gas sensor unit of the present invention, the filter is disposed out of view through any of the introduction passages. Therefore, even when water enters at high speed from the outside through a certain introduction passage, the high-speed water does not directly hit the filter. That is, water having entered at high speed from the outside through the introduction passage is interrupted by a wall surface of the casing before reaching the filter, whereby the flow strength (velocity) of water can be reduced. Thus, it becomes possible to further reduce the possibility that water having entered from the outside through the introduction passages passes through the filter. Similarly, it becomes possible to further reduce the possibility that the filter is damaged by water or a foreign object (sand particles or the like) having entered from the outside through the introduction passages. Moreover, even when an operator accidentally inserts an attachment tool or the like into the interior of the casing through the introduction passages, the attachment tool or the like is unlikely to reach the filter, so that the possibility of the filter being damaged is small.

By virtue of the above-described configuration, the gas sensor unit of the present invention can reduce the possibility that water having entered from the outside via the introduction passages passes through the filter, while improving the responsiveness of the gas sensor. Further, the gas sensor unit can reduce the possibility that the filter is damaged by a foreign object (sand particles, attachment tool, or the like) having entered from the outside through the introduction passages, while improving the responsiveness of the gas sensor.

In the above-described gas sensor unit, each of the plurality of introduction passages is preferably disposed to face one of the remaining introduction passages via the pre-filter passage.

In the gas sensor unit according to a preferred embodiment of the present invention, each of the plurality of introduction passages is disposed to face one of the remaining introduction passages via the pre-filter passage. This arrangement increases the possibility that water having entered at high speed into the casing via a certain introduction passage is directly discharged to the outside via another introduction passage opposite the certain introduction passage. Accordingly, the gas sensor unit of the present invention can further reduce the possibility that water having entered the gas flow passage via the introduction passages remains within the pre-filter passage, or that the pressure of water exceeds the withstand water pressure of the filter and the water passes through the filter.

In any of the above-described gas sensor units, preferably, the casing has a plurality of introduction wall surfaces which respectively define the plurality of introduction passages, and at least one of the introduction wall surfaces has a configuration such that the distance, with respect to the thickness direction of the filter, between the filter and a filter-side introduction wall surface, which is a surface section of the introduction wall surface located on a side toward the filter with respect to the thickness direction of the filter, gradually increases from an outer end to an inner end of the corresponding introduction passage.

In the gas sensor unit according to a preferred embodiment of the present invention, at least one of the filter-side introduction wall surfaces is configured such that the distance between the filter-side introduction wall surface and the filter with respect to the thickness direction of the filter gradually increases from an outer end to an inner end of the corresponding introduction passage. Such a filter-side introduction wall surface can control the flow direction of water, which has entered at high speed and hit the filter-side introduction wall surface, for moving further away from the filter, as compared to the case where the distance between the filter-side introduction wall surface and the filter with respect to the thickness direction of the filter does not change.

This configuration further reduces the possibility that water bearing a higher pressure than the withstand water pressure of the filter enters from the outside via the introduction passage and passes through the filter. Further, the configuration further reduces the possibility that the filter is damaged by a foreign object or the like having entered from the outside through the introduction passages. Moreover, even when an operator accidentally inserts an attachment tool or the like into the interior of the casing through the introduction passages, the attachment tool or the like does not reach the filter, so that the possibility of the filter being damaged is further reduced.

Notably, the filter-side introduction wall surface, which is configured such that the distance between the filter-side introduction wall surface and the filter with respect to the thickness direction of the filter gradually increases from the outer end to the inner end of the corresponding introduction passage, may be a flat surface inclined at a predetermined angle or a curved surface.

In the above-described gas sensor unit, preferably, an opposite-side introduction wall surface, which is a surface section of the introduction wall surface located opposite the filter-side introduction wall surface, is configured such that the distance between the opposite-side introduction wall surface and the filter-side introduction wall surface gradually increases from the inner end to the outer end of the corresponding introduction passage.

In the gas sensor unit according to a preferred embodiment of the present invention, the opposite-side introduction wall surface of an introduction wall surface that defines an introduction passage is configured to separate away from the filter-side introduction wall surface, from the inner end to the outer end of the corresponding introduction passage. Therefore, when the size of the inner opening of the introduction passage is maintained constant, the size of the outer opening of the introduction passage can be increased, as compared with the case where the opposite-side introduction wall surface is parallel to the filter-side introduction wall surface. Accordingly, ambient gas can be easily introduced into the interior of the casing (into the gas flow passage) via the introduction passage. Thus, the responsiveness of the gas sensor can be further improved.

Notably, the opposite-side introduction wall surface may assume any configuration, so long as the opposite-side introduction wall surface is configured to gradually separate away from the filter-side introduction wall surface, from the inner end to the outer end of the corresponding introduction passage. That is, the opposite-side introduction wall surface may be configured such that the distance between the opposite-side introduction wall surface and the filter with respect to the thickness direction of the filter gradually increases, from the outer end to the inner end of the corresponding introduction passage, may be configured such that the distance gradually decreases, from the outer end to the inner end of the corresponding introduction passage, or may be configured such that the distance is constant. Further, the opposite-side introduction wall surface may be a flat surface inclined at a predetermined angle, or a curved surface.

Moreover, in any one of the above-described gas sensor units, preferably, the casing has a plurality of introduction wall surfaces which respectively define the plurality of introduction passages, and a filter holding portion of the casing for holding the filter and the plurality of introduction wall surfaces are molded integrally.

As compared with the case where the filter holding portion (a member having the filter holding portion) and the introduction wall surfaces (a member having the introduction wall surfaces) are formed of separate members, the number of components and the number of manufacturing steps can be reduced, whereby the gas sensor unit can be made inexpensive.

Furthermore, the present invention is also directed to a method for providing the gas sensor unit as described herein. Embodiments of the present invention will be described with respect to the following drawings:
FIG. 1 shows a top view of a gas sensor unit 100 according to Embodiment 1.
FIG. 2 shows a side view of the gas sensor unit 100 according to Embodiment 1, corresponding to a view as viewed in the direction of arrow B of FIG. 1.
FIG. 3 is a view showing a main portion of the gas sensor unit 100 according to Embodiment 1, corresponding to a sectional view taken along line C-C of FIG. 1.
FIG. 4 shows an enlarged sectional view showing the introduction passage S1 and its vicinity of the gas sensor unit 100 of Embodiment 1.
FIG. 5 shows an explanatory view showing a water resistance test for the gas sensor unit 100 of Embodiment 1.

A gas sensor unit 100 according to Embodiment 1 will be described with reference to the drawings.

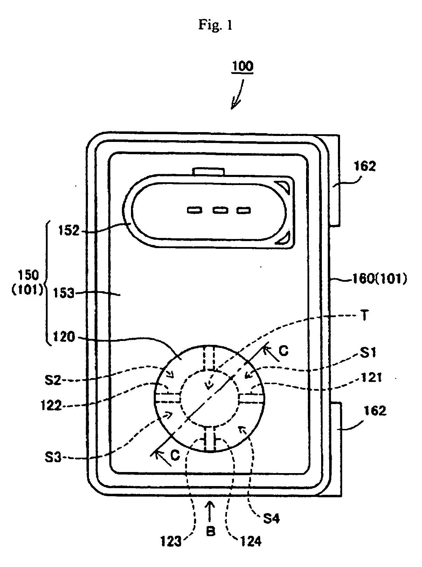

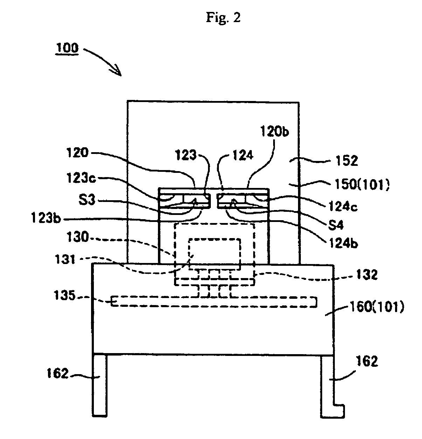

FIG. 1 is a top view of the gas sensor unit 100 of Embodiment 1, and FIG. 2 is its side view, which corresponds to a view as viewed in the direction of arrow B of FIG. 1. As shown in FIGS. 1 and 2, the gas sensor unit 100 includes a first casing member 150, a second casing member 160, a gas sensor 130, and a circuit board 135. The gas sensor 130 and the circuit board 135 are accommodated within a casing 101, which is composed of the first casing member 150 and the second casing member 160 integrally assembled.

The first casing 150 is integrally molded by use of resin and assumes the shape of a cover. The first casing 150 includes a gas sensor accommodation portion 120 for accommodating the gas sensor 130, a terminal connector section 152 for electrical connection with an external apparatus, and a cover portion 153 fitted into the second casing member 160 so as to close the opening of the second casing member 160. Of these portions, the gas sensor accommodation portion 120 has introduction passages S1 to S4 formed by means of introduction wall surfaces 121 to 124. Thus, external air (atmospheric gas) can be introduced into the interior of the casing 101 via the introduction passages S1 to S4.

The second casing 160 is integrally molded by use of resin and assumes the shape of a box. The second casing 160 includes attachment portions 162 for attaching the gas sensor unit 100 to the body of an automobile, a duct of an air conditioner for a vehicle, or the like.

The circuit board 135 is formed of glass cloth base epoxy resin, and supports electronic components such as the gas sensor 130.

The gas sensor 130 includes a gas sensor element 131, and a sleeve 132 surrounding the gas sensor element 131 and having a gas introduction hole. The gas sensor element 131 includes first and second gas-sensitive bodies and an electric heater element, which are mounted on an alumina substrate. The first gas-sensitive body is a body (e.g., a body mainly formed of WO₃) whose resistance changes with concentration of an oxidizing gas such as NOₓ. The second gas-sensitive body is a body (e.g., a body mainly formed of SnO₂) whose resistance changes with concentration of a reducing gas such as HC or CO. The electric heater heats the first and second gas-sensitive bodies. Notably, in the present embodiment (Embodiment 1), a gas sensor of Figaro Engineering Inc. (No. TGS2201) is used as the gas sensor 130.

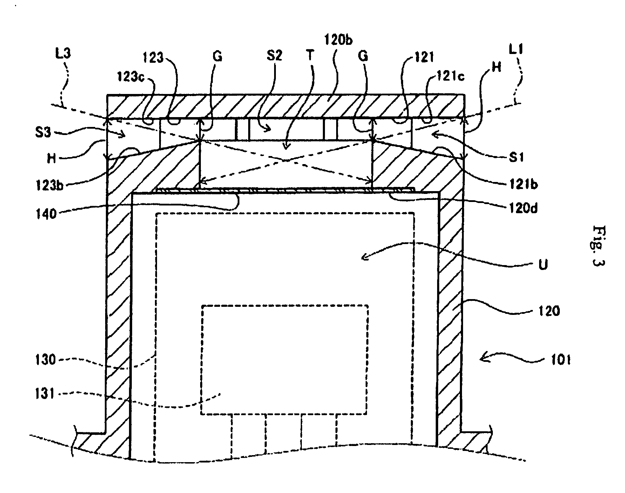

FIG. 3 shows a side sectional view of the gas sensor accommodation portion 120, taken along line C-C of FIG. 1.

As shown in FIG. 3, the casing 101 (the gas sensor accommodation portion 120) forms a gas flow passage for guiding to the gas sensor element 131 external air (atmospheric gas) introduced from the outside. Specifically, this gas flow passage is composed of the introduction passages S1 to S4, a pre-filter passage T connected with each of the introduction passages S1 to S4, a filter 140 located at a bottom end of the pre-filter passage T, and a post-filter space U located between the filter 140 and the gas sensor element 131.

As shown in FIG. 1, each of the introduction passages S1 to S4 assumes the shape of a quadrant of a circular ring as viewed from above and is adjacent to the outside of the casing 101. The pre-filter passage T is a cylindrical space between a ceiling portion 120b of the gas sensor accommodation portion 120, and the filter 140. The introduction passages S1 to S4 are disposed around the pre-filter passage T to be located adjacent thereto. Therefore, external air (atmospheric gas) entering the gas flow passage via the introduction passages S1 to S4 is quickly introduced to the pre-filter passage T irrespective of the position of the introduction passage S1, S2, S3 or S4 though which the external air passes.

As shown in FIG. 3, the pre-filter passage T is disposed such that the filter 140 faces the pre-filter passage T. In other words, the filter 140 is provided at a location where the filter 140 faces the pre-filter passage T, and nothing is disposed between the filter 140 and the pre-filter passage T. Therefore, as compared with the case where, in order to prevent entry of water, a barrier wall is provided between the introduction passages S1 to S4 and the filter 140 such that the pre-filter passage T is bent to form a maze-like flow path, external air (atmospheric gas) entering from the outside can be quickly led from the pre-filter passage T to the gas sensor element 131 via the filter 140, so that the gas sensor can have excellent responsiveness.

The sheet-shaped filter 140, which has gas permeability and water repellency, is affixed to a filter holding portion 120d of the gas sensor accommodation portion 120, so as to water-tightly close the gas flow passage at the middle thereof while allowing passage of air therethrough. This structure enables external air introduced from the outside via the introduction passages S1 to S4 to be led to the gas sensor element 131, and prevents water having entered the gas flow passage via the introduction passages S1 to S4 from reaching the gas sensor element 131 and the circuit board 135, unless the pressure of water exceeds the withstand water pressure of the filter 140.

The filter 140 has a porous fiber structure formed of, for example, polytetrafluoroethylene. In the present embodiment (Embodiment 1), the filter 140 is formed of Gore-Tex (trademark) of Japan Gore-Tex Inc. (product name: oleo vent filter; model number: S-type; withstand water pressure: 19.6 kPa or higher).

In the present embodiment (Embodiment 1), an oil repellant agent is applied to the surface of the filter 140, so that the filter 140 has oil repellency as well as gas permeability and water repellency. The upper surface of a peripheral portion of the filter 140 is welded to the filter holding portion 120d of the gas sensor accommodation portion 120 by means of ultrasonic welding or impulse welding, whereby the filter 140 is affixed to the filter holding portion 120d.

In the gas sensor unit 100 of the present embodiment (Embodiment 1), as shown in FIG. 3, the filter holding portion 120d and the introduction wall surfaces 121 to 124 that constitute the introduction passages S1 to S4 are provided on the first casing member 150 (gas sensor accommodation portion 120). That is, the filter holding portion 120d and the introduction wall surfaces 121 to 124 that constitute the introduction passages S1 to S4 are integrally molded. Therefore, as compared with the case where a filter holding portion (a member having the filter holding portion) and introduction wall surfaces (a member having the introduction wall surfaces) are formed of separate members, the number of components and the number of manufacturing steps can be reduced, whereby the gas sensor unit can be made inexpensive.

Incidentally, as can be seen from FIGS. 1 and 3, the introduction passages S1 and S3 are disposed at respective positions such that one of the introduction passages can be viewed from the outside of the casing 101 via the other introduction passage and the pre-filter passage T. Specifically, the introduction passages S1 and S3 are disposed at opposite locations with respect to the pre-filter passage T. Similarly, the introduction passages S2 and S4 are disposed at opposite locations with respect to the pre-filter passage T. By virtue of such a structure, even when water enters from the outside at high speed via a certain one of the introduction passages S1 to S4, the water having entered can be discharged to the outside via another of the introduction passages S1 to S4 located opposite the certain introduction passage.

Specifically, for example, when water enters from the outside via the introduction passage S1 at high speed such that the water flows in a direction from the introduction passage S1 toward the introduction passage S3 (leftward in FIG. 3), the water having entered at high speed can be discharged to the outside from the introduction passage S3 via the pre-filter passage T. Accordingly, the water having entered the gas flow passage (interior of the casing 101) via the introduction passages S1 to S4 can be prevented from remaining within the pre-filter passage T, thereby reducing the possibility that the pressure of water exceeds the withstand water pressure of the filter 140 and the water passes through the filter 140.

Further, as can be understood from an arrow L1 shown in FIG. 3 (the arrow corresponding to a line of vision at the time one views a point closest to the filter 140 (lower side in FIG. 3) from the outside via the introduction passage S1), the filter 140 cannot be seen from the outside via the introduction passage S1. Similarly, as can be understood from a line of vision L3 shown in FIG. 3 (corresponding to a line of vision at the time one views a point closest to the filter 140 (lower side in FIG. 3) from the outside via the introduction passage S3), the filter 140 cannot be seen from the outside via the introduction passage S3. Moreover, although not illustrated in the drawing, the filter 140 cannot be seen from the outside via the introduction passage S2 or S4. That is, in the gas sensor unit 100 of Embodiment 1, the filter 140 is disposed at a location which cannot be seen from the outside via any of the introduction passages S1 to S4.

Therefore, even when water enters at high speed from the outside through any of the introduction passages S1 to S4, the high-speed water does not directly hit the filter 140. That is, water having entered at high speed from the outside through any of the introduction passages S1 to S4 cannot reach the filter 140 without hitting the inner wall surface of the casing 101 (the gas sensor accommodation portion 120), whereby the flow strength (velocity) of water can be reduced. Thus, it becomes possible to further reduce the possibility that water having entered from the outside through the introduction passages S1 to S4 passes through the filter 140. Similarly, it becomes possible to further reduce the possibility that the filter 140 is damaged by water or a foreign object (sand particles or the like) having entered from the outside through the introduction passages S1 to S4. Moreover, even when an operator accidentally inserts an attachment tool or the like into the interior of the casing 101 through the introduction passages S1 to S4, the attachment tool or the like does not reach the filter 140, so that the possibility of the filter 140 being damaged is small.

Here, of the introduction wall surfaces 121 to 124, which define the introduction passages S1 to S4, wall surface sections located on the side toward the filter 140 (lower side in FIG. 3) as viewed in the thickness direction of the filter 140 (vertical direction in FIG. 3), are referred to as filter-side introduction wall surfaces 121b to 124b. further, wall surface sections located on the side opposite the filter-side introduction wall surfaces 121b to 124b (upper side in FIG. 3) are referred to as opposite-side introduction wall surfaces 121c to 124c.

As shown in FIG. 3, the filter-side introduction wall surface 121b is configured such that the distance between this wall surface and the filter 140 with respect to the thickness direction of the filter 140 (vertical direction in FIG. 3) gradually increases from the outer end of the introduction passage S1 toward the inner end thereof (leftward in FIG. 3). Specifically, the filter-side introduction wall surface 121b is a surface inclined at a predetermined angle (10.6° in Embodiment 1) such that the vertical position of the surface gradually moves upward in FIG. 3 from the outer end of the introduction passage S1 toward the inner end thereof. Similarly, the filter-side introduction wall surface 123b is configured such that the distance between this wall surface and the filter 140 with respect to the thickness direction of the filter 140 (vertical direction in FIG. 3) gradually increases from the outer end of the introduction passage S3 toward the inner end thereof (toward the rightward end in FIG. 3). Moreover, although not illustrated in the drawings, the filter-side introduction wall surfaces 122b and 124b are configured in a similar manner.

Here, a state in which water enters the introduction passages S1 to S4 at high speed from the outside, and hits the filter-side introduction wall surfaces 121b to 124b, will be described, while the filter-side introduction wall surface 121b is taken as an example. Here, the state will be described in contrast to the case where the distance between each filter-side introduction wall surface and the filter 140 as measured in the thickness direction of the filter 140 does not change (each filter-side introduction wall surface is in parallel to the permeation surface of the filter 140).

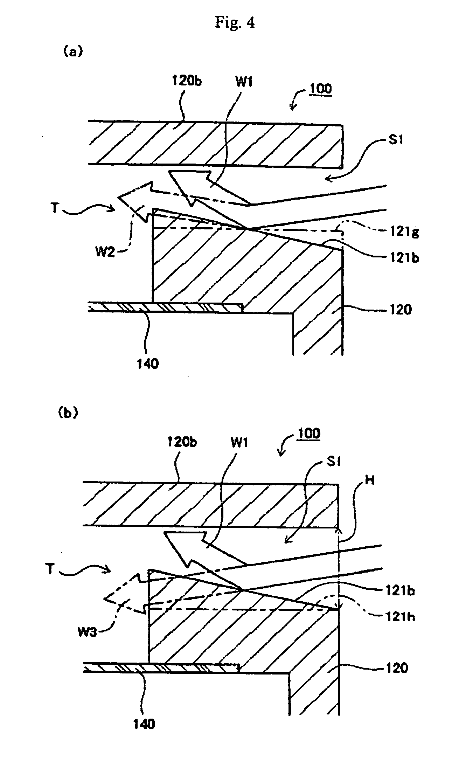

FIG. 4 is an enlarged sectional view showing the introduction passage S1 (filter-side introduction wall surface 121b) and its vicinity of the gas sensor unit 100 of Embodiment 1. As shown in FIG. 4(a), the gas sensor unit 100 of Embodiment 1 can cause water having entered the introduction passage S1 at high speed to hit the filter-side introduction wall surface 121b, and changes the flow direction of the water toward a direction for flowing away from the filter 140 as indicated by arrow W1, while reducing the flow strength (velocity) of the water having entered at high speed. Here, the above condition is compared with the case indicated by an imaginary line in FIG. 4(a), where the filter-side introduction wall surface is parallel to the permeation surface of the filter 140 (the filter-side introduction wall surface in this case will be referred to as the filter-side introduction wall surface 121g). In this case, water having entered the introduction passage S1 at high speed hits the filter-side introduction wall surface 121g, and then advances in the direction indicated by arrow W2. As is apparent from comparison between arrows W1 and W2, the filter-side introduction wall surface 121b of Embodiment 1 can change the flow direction of water toward a direction (upper direction in the drawing) for flowing further away from the filter 140 as compared with the case of the filter-side introduction wall surface 121g.

Further, the present embodiment is compared with a case where, as shown in FIG. 4(b), the filter-side introduction wall surface is parallel to the permeation surface of the filter 140 with the size of the outside opening H being rendered the same as that in Embodiment 1 (the filter-side introduction wall surface in this case will be referred to as the filter-side introduction wall surface 121h). In this case, as indicated by arrow W3, water having entered the introduction passage S1 at high speed is likely to enter the pre-filter passage T without hitting the filter-side introduction wall surface 121h. Accordingly, through employment of the configuration having the filter-side introduction wall surface 121b rather than the configuration having the filter-side introduction wall surface 121h, the possibility that the pressure of water having entered via the introduction passage S1 exceeds the withstand water pressure of the filter 140 and the water passes through the filter 140 is lowered.

In the gas sensor unit 100 of Embodiment 1, the filter-side introduction wall surfaces 121b to 124b are formed such that the distances between the wall surfaces and the filter 140 with respect to the thickness direction of the filter 140 gradually increase from the outer ends toward the inner ends of the introduction passages S1 to S4. Therefore, the possibility that the pressure of water having entered via the introduction passages S1 to S4 exceeds the withstand water pressure of the filter 140 and the water passes through the filter 140 can be further reduced. Further, the possibility that the filter 140 is damaged by a foreign object or the like having entered via the introduction passages S1 to S4 can be further reduced.

In addition, in the gas sensor unit 100 of Embodiment 1, as shown in FIG. 3, the opposite-side introduction wall surfaces 121c and 123c are configured such that the distances between these wall surfaces and the corresponding filter-side introduction wall surfaces 121b and 123b gradually increase from the inner ends toward the outer ends of the introduction passages S1 and S3. Further, although not illustrated, the opposite-side introduction wall surfaces 122c and 124c are configured in the same manner. Through employment of these configurations, when the size of an inner opening G is maintained constant, the size of an outer opening H can be increased, as compared with, for example, the case where the opposite-side introduction wall surfaces 121c to 124c are parallel to the filter-side introduction wall surfaces 121b to 124b. Accordingly, in the gas sensor unit 100 of Embodiment 1, conceivably, external ambient gas can be easily introduced into the interior of the casing 101 (into the gas flow passage) via the introduction passages S1 to S4. Thus, the responsiveness of the gas sensor can be further improved.

In the present embodiment (Embodiment 1), a water resistance test based on DIN 40050 IP 9K was performed in order to evaluate the water resistance of the gas sensor unit 100.

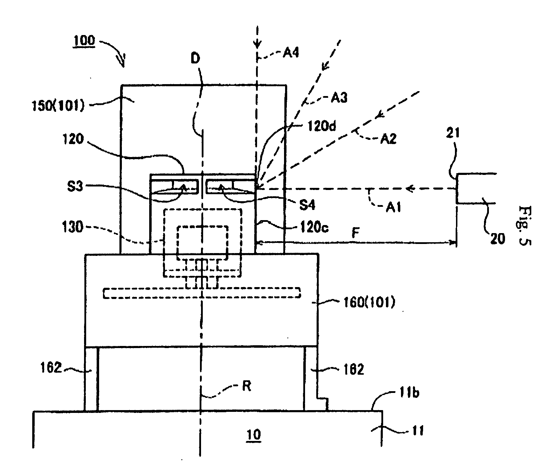

Specifically, as shown in FIG. 5, a rotary jig 10 including a rotary plate 11 having a horizontal mount surface 11b is first prepared, and the gas sensor unit 100 is placed on the mount surface 11b. Subsequently, the gas sensor unit 100 is fixed to the rotary plate 11 by use of the attachment portions 162 such that the vertically extending rotary axis R of the rotary plate 11 and the center axis D of the gas sensor accommodation portion 120 of the gas sensor unit 100 coincide with each other.

Further, a high-pressure water discharge unit 20 is disposed on a side of the gas sensor unit 100. Specifically, as shown in FIG. 5, a discharge port 21 of the high-pressure water discharge unit 20 is positioned at a location which is horizontally separated by a distance F (= 100 mm in Embodiment 1) from an introduction opening 120e on a side surface 120c of the gas sensor accommodation portion 120 at which the introduction passages S1 to S4 are formed. The discharge angle A1 at which water is discharged toward the gas sensor unit 100 from the discharge port 21 disposed at the above-described location is defined to be zero. Notably, the high-pressure water discharge unit 20 is configured to change the discharge angle within a range of 0° to 90° (angles between A1 to A4 in the drawing), while maintaining the distance F between the discharge port 21 and the introduction opening 120e at 100 mm.

Subsequently, the rotary plate 11 was rotated about the rotary axis G at a rotational speed of 5 turns/min, and warm water (80°C) was discharged from the discharge port 21 under a discharge pressure of 8 MPa at the discharge angle A1 (0°). While the distance F between the discharge port 21 and the introduction opening 120e was maintained at 100 mm, the discharge angle was changed among A1, A2, A3, and A4 (0°, 30°, 60°, and 90°) in this order, and at each position, water was continuously discharged for 30 seconds.

Subsequently, the gas sensor unit 100 was removed from the rotary jig 10, and was disassembled so as to check the interior thereof. Although a slight quantity of water entered the introduction passages S1 to S4 and the pre-filter passage T, no water reached the gas sensor 130 (the gas sensor element 131). From this result, it can be said that in the gas sensor unit 100 of Embodiment 1, water having entered via the introduction passages S1 to S4 is unlikely to pass through the filter 140.

In the above, Embodiment 1 of the present invention has been described. However, the present invention is not limited to the above-described embodiment and the like, and, needless to say, can be applied with appropriate modifications without departing from the scope of the present invention.

For example, the number, position, shape, size, etc. of the introduction passages S1, etc. can be freely changed in accordance with the mounting position and required performances of the gas sensor unit 100 and in consideration of the responsiveness, water resistance, etc. of the gas sensor.

In the gas sensor unit 100 of Embodiment 1, all the introduction passages S1 to S4 are configured such that the distances between the filter-side introduction wall surfaces 121b to 124b and the filter 140 with respect to the thickness direction of the filter 140 (vertical direction in FIG. 3) gradually increase from the outer ends toward the inner ends of the introduction passages S1 to S4. However, the configurations of the filter-side introduction wall surfaces 121b to 124b may be freely changed in consideration of the responsiveness, water resistance, etc. of the gas sensor. For example, in the gas sensor unit 100 of Embodiment 1, since the terminal connector section 152 stands adjacent to the gas sensor accommodation portion 120 in which the introduction passages S1 to S4 are formed, water is less likely to enter through the introduction passages S1 and S2 provided on the side toward the terminal connector portion 152. Therefore, the filter-side introduction wall surfaces 121b and 122b, which constitute the introduction passages S1 and S2, may be formed to have configurations different from the above-described configurations, in consideration of the responsiveness, etc. of the gas sensor.

In the gas sensor unit 100 of Embodiment 1, the filter-side introduction wall surfaces 121b to 124b are inclined surfaces (flat surfaces) of a constant angle whose vertical positions gradually move upward in FIG. 3 from the outer ends toward the inner ends of the introduction passages S1 to S4. However, the filter-side introduction wall surfaces 121b to 124b may be curved surfaces. Irrespective of whether the filter-side introduction wall surfaces 121b to 124b are flat surfaces or curved surfaces, the filter-side introduction wall surfaces 121b to 124b, which are configured such that the distances between these wall surfaces and the filter 140 with respect to the thickness direction of the filter 140 gradually increase from the outer ends toward the inner ends of the introduction passages S1, etc., further reduce the possibility that the pressure of water having entered via the introduction passages S1, etc. exceeds the withstand water pressure of the filter 140 and the water passes through the filter 140. Further, the possibility that the filter 140 is damaged by a foreign object or the like having entered via the introduction passages S1, etc. can be further reduced.

### Reference Numbers

100: gas sensor unit
120: gas sensor accommodation portion
121, 122, 123, 124: introduction wall surfaces
121b, 122b, 123b, 124b: filter-side introduction wall surfaces
121c, 122c, 123c, 124c: opposite-side introduction wall surfaces
130: gas sensor
131: gas sensor element
140: filter
101: casing
S1, S2, S3, S4: introduction passages
T: pre-filter passage
U: post-filter space

## Claims

1. A gas sensor unit (100) comprising:
a gas sensor element (131) for detecting changes in concentration of a specific gas contained in ambient gas;
a casing (101) for accommodating the gas sensor element, and forming a gas flow passage for leading to the gas sensor element the ambient gas introduced from the outside of the casing;
a filter (140) having gas permeability and water repellency and water-tightly closing the gas flow passage at the middle thereof while allowing passage of gas, wherein
the gas flow passage includes a plurality of introduction passages (S1,S2,S3,S4) for introducing the ambient gas from the outside of the casing, and a pre-filter passage (T) which is located between the introduction passages and the filter (140) and which the filter faces; and
the plurality of introduction passages are disposed at respective positions such that each introduction passage (S1,S2,S3,S4) enables viewing of at least a portion of another introduction passage from the outside of the casing via the former introduction passage and the pre-filter passage (T), wherein each of the plurality of introduction passages (S1,S2,S3,S4) is disposed to face one of the remaining introduction passages via the pre-filter passage (T);
wherein the casing (101) has a plurality of introduction wall surfaces (121b,121c, 122b,122c, 123b,123c, 124b, 124c) which respectively define the plurality of introduction passages, wherein the introduction wall surfaces comprises ; filter-side introduction wall surfaces (121b,122b,123b,124b);
**characterized in that**
the filter is disposed at a position which cannot be viewed from the outside of the casing via any of the introduction passages,
and each of the filter-side introduction wall surfaces has a configuration such that the distance, with respect to the thickness direction of the filter (140), between the filter and the respective filter-side introduction wall surface (121b,122b,123b,124b), which is a surface section of the introduction wall surface located on a side toward the filter with respect to the thickness direction of the filter, gradually increases from an outer end to an inner end of the corresponding introduction passage.

2. A gas sensor unit according to claim 1, wherein an opposite-side introduction wall surface (121b,122b,123b,124b), which is a surface section of the introduction wall surface located opposite the filter-side introduction wall surface (121b,122b,123b,124b), is configured such that the distance between the opposite-side introduction wall surface and the filter-side introduction wall surface gradually increases from the inner end to the outer end of the corresponding introduction passage.

3. A gas sensor unit according to claim 1 or 2, wherein the casing (101) has a plurality of introduction wall surfaces which respectively define the plurality of introduction passages, and a filter holding portion (120d) of the casing for holding the filter and the plurality of introduction wall surfaces are molded integrally.

## Patentansprüche

1. Eine Gassensoreinheit (100), umfassend:
ein Gassensorelement (131) zum Detektieren von Konzentrationsänderungen von einem in einem Umgebungsgas enthaltenen spezifischen Gas;
ein Gehäuse (101) zum Aufnehmen des Gassensorelements und Ausbilden eines Gasströmungskanals zum Führen des Umgebungsgases von dem Äußeren des Gehäuses zu dem Gassensorelement;
einen Filter (140) mit Gasdurchlässigkeit und Wasserabweisungsvermögen und den Gasströmungskanal in dessen Mitte wasserdicht verschließend während ein Gasdurchgang gewährleistet wird,
wobei der Gasströmungskanal eine Mehrzahl von Einleitungspassagen (S1, S2, S3, S4) zum Einleiten des Umgebungsgases von dem Äußeren des Gehäuses und eine Vorfilterpassage (T) umfasst, die zwischen den Einleitungspassagen und dem Filter (140) angeordnet ist und dem Filter gegenüberliegt; und
wobei die Mehrzahl von Einleitungspassagen an entsprechenden Positionen angeordnet sind, so dass jede Einleitungspassage (S1, S2, S3, S4) ein Betrachten von wenigstens einem Bereich von einer weiteren Einleitungspassage vom Äußeren des Gehäuses via der vorherigen Einleitungspassage und der Vorfilterpassage (T) ermöglicht, wobei jede der Mehrzahl von Einleitungspassagen (S1, S2, S3, S4) so angeordnet ist, dass sie einer der restlichen Einleitungspassagen via der Vorfilterpassage (T) gegenüberliegt;
wobei das Gehäuse (101) eine Mehrzahl von Einleitungswandoberflächen (121b, 121c, 122b, 122c, 123b, 123c, 124b, 124c) umfasst, welche jeweils die Mehrzahl an Einleitungspassagen definieren, wobei die Einleitungswandoberflächen filterseitige Einleitungswandoberflächen (121b, 122b, 123b, 124b) umfassen; **dadurch gekennzeichnet, dass**
der Filter an einer Position angeordnet ist, welche vom Äußeren des Gehäuses nicht via irgendeiner der Einleitungspassagen betrachtet werden kann, und jede der filterseitigen Einleitungswandoberflächen eine Konfiguration aufweist, sodass der Abstand in Bezug auf die Dickenrichtung des Filters (140) zwischen dem Filter und der entsprechenden filterseitigen Einleitungswandoberfläche (121 b, 122b, 123b, 124b), welche ein Oberflächenabschnitt der Einleitungswandoberfläche ist, welcher in Bezug zu der Dickenrichtung des Filters an einer zum Filter gewandten Seite angeordnet ist, von einem äußeren Ende zu einem inneren Ende der entsprechenden Einleitungspassage graduell zunimmt.

2. Eine Gassensoreinheit gemäß Anspruch 1, wobei eine gegenüberliegende Einleitungswandoberfläche (121 b, 122b, 123b, 124b), welche einen Oberflächenabschnitt der Einleitungswandoberfläche darstellt, die gegenüber der filterseitigen Einleitungswandoberfläche (121 b, 122b, 123b, 124b) angeordnet ist, so konfiguriert ist, dass der Abstand zwischen der gegenüberliegende Einleitungswandoberfläche und der filterseitigen Einleitungswandoberfläche von dem inneren Ende zu dem äußeren Ende der entsprechenden Einleitungspassage graduell zunimmt.

3. Eine Gassensoreinheit gemäß Anspruch 1 oder 2, wobei das Gehäuse (101) eine Mehrzahl von Einleitungswandoberflächen, welche jeweils die Mehrzahl von Einleitungspassagen definieren, und einen Filterhaltebereich (120d) des Gehäuses zum Halten des Filters umfasst, und wobei die Mehrzahl von Einleitungswandoberflächen integral ausgeformt sind.

## Revendications

1. Détecteur de gaz (100) qui comprend :
un détecteur de gaz (131) destiné à détecter les changements de concentration en gaz spécifique contenu dans un gaz ambiant ;
un boîtier (101) destiné à contenir ledit détecteur de gaz, et formant un passage d'écoulement de gaz destiné à acheminer vers ledit détecteur de gaz le gaz ambiant introduit depuis l'extérieur dudit boîtier ;
un filtre (140) ayant une perméabilité au gaz et un pouvoir hydrofuge, et fermant de manière étanche à l'eau ledit passage d'écoulement de gaz au milieu de celui-ci, tout en permettant le passage du gaz, dans lequel
ledit passage d'écoulement de gaz comprend une pluralité de passages d'introduction (S1, S2, S3, S4) destinés à introduire ledit gaz ambiant provenant de l'extérieur dudit boîtier, et un passage de pré-filtrage (T) qui est situé entre lesdits passages d'introduction et ledit filtre (140) et qui fait face audit filtre ; et
ladite pluralité de passages d'introduction est disposée à des emplacements respectifs de sorte que chaque passage d'introduction (S1, S2, S3, S4) permette de voir au moins une partie d'un autre passage d'introduction depuis l'extérieur dudit boîtier via le passage d'introduction précédent et ledit passage de pré-filtrage (T), dans lequel chacun de ladite pluralité de passages d'introduction (S1, S2, S3, S4) est disposé pour faire face à l'un des passages d'introduction restants via ledit passage de pré-filtrage (T) ; dans lequel ledit boîtier (101) possède une pluralité de surfaces de parois d'introduction (121b, 121c, 122b, 122c, 123b, 123c, 124b, 124c) qui définissent respectivement ladite pluralité de passages d'introduction, lesdites surfaces de parois d'introduction comprenant des surfaces de parois d'introduction côté filtre (121b, 122b, 123b, 124b) ; **caractérisé en ce que**
ledit filtre est disposé à un emplacement qui ne peut être vu depuis l'extérieur dudit boîtier via un quelconque desdits passages d'introduction, et chacune desdites surfaces de parois d'introduction côté filtre possède une configuration telle que la distance par rapport à l'épaisseur dudit filtre (140), entre ledit filtre et la surface de paroi d'introduction respective côté filtre (121b, 122b, 123b, 124b), qui est une section de surface de la surface de paroi d'introduction qui se trouve sur un côté tourné vers ledit filtre par rapport à l'épaisseur dudit filtre, augmente progressivement d'une extrémité extérieure vers une extrémité intérieure du passage d'introduction correspondant.

2. Détecteur de gaz selon la revendication 1, dans lequel une surface de paroi d'introduction de côté opposé (121b, 122b, 123b, 124b), qui est une section de surface de la surface de paroi d'introduction opposée à la surface de paroi d'introduction côté filtre (121b, 122b, 123b, 124b), est configurée de sorte que la distance entre la surface de paroi d'introduction du côté opposé et la surface de paroi d'introduction côté filtre augmente progressivement entre l'extrémité intérieure et l'extrémité extérieure du passage d'introduction correspondant.

3. Détecteur de gaz selon la revendication 1 ou 2, dans lequel ledit boîtier (101) possède une pluralité de surfaces de parois d'introduction qui définissent respectivement ladite pluralité de passages d'introduction, et une partie de support de filtre (120d) dudit boîtier destinée à supporter ledit filtre et une pluralité de surfaces de parois d'introduction sont moulées intégralement.
